# EUROPEAN PATENT SPECIFICATION

(11) **EP 2 934 198 B1**
(45) Date of publication and mention of the grant of the patent: **22.04.2020**
(21) Application number: 13823946.2
(22) Date of filing: 16.12.2013
(51) Int. Cl.: A24B 15/16, A24B 15/28, A61M 11/04, A61M 15/06, A24F 47/00

(54) **ENCAPSULATED VOLATILE LIQUID SOURCE FOR AN AEROSOL-GENERATING SYSTEM**
VERKAPSELTE FLÜCHTIGE FLÜSSIGKEITSQUELLE FÜR EIN AEROSOL-ERZEUGUNGSSYSTEM
SOURCE DE LIQUIDE VOLATILE ENCAPSULÉE POUR UN SYSTÈME DE GÉNÉRATION D'AÉROSOL

(30) Priority: 18.12.2012 EP 12197848
(43) Date of publication of application: 28.10.2015
(73) Proprietor: Philip Morris Products S.A., 2000 Neuchâtel (CH)
(72) Inventor: WALLER, Judith, CH-2034 Peseux (CH); MOOR, Philippe, CH-2063 Fenin (CH)
(74) Representative: Millburn, Julie Elizabeth
(86) International application number: PCT/EP2013/076673
(87) International publication number: WO 2014/095701

(56) References cited:
- WO-A1-2010/107613
- US-A1- 2012 298 123

## Description

The present invention relates to an aerosol-generating system comprising an encapsulated volatile liquid source. In particular, the present invention relates to an aerosol-generating system comprising an encapsulated volatile liquid source for generating an aerosol comprising nicotine salt particles.

WO 2008/121610 A1, WO 2010/107613 A1 and WO 2011/034723 A1 disclose devices and methods for delivering nicotine or another medicament to a user. The devices comprise a volatile acid source or another volatile delivery enhancing compound source and a nicotine source or another medicament source. In preferred embodiments, pyruvic acid is reacted with nicotine in the gas phase to form an aerosol of nicotine pyruvate salt particles that is inhaled by the user.

A sorption element with a volatile liquid sorbed thereon will have a tendency to lose volatile liquid when stored for any length of time. In devices of the type disclosed in WO 2008/121610 A1, WO 2010/107613 A1 and WO 2011/034723 A1 it is desirable to retain sufficient volatile delivery enhancing compound and sufficient nicotine or another medicament during storage to generate a desired aerosol in use. It is also desirable to retain the volatile delivery enhancing compound and the nicotine or another medicament during storage without degradation by oxidation, hydrolysis or other unwanted reactions, which may alter the properties of the reactants.

It would be desirable to provide an aerosol-generating system for delivering nicotine or another medicament to a user of the type disclosed in WO 2008/121610 A1, WO 2010/107613 A1 and WO 2011/034723 A1 in which the retention of one or both of the volatile delivery enhancing compound and the nicotine or another medicament during storage is improved. It would also be desirable to provide an aerosol-generating system for delivering nicotine or another medicament to a user of the type disclosed in WO 2008/121610 A1, WO 2010/107613 A1 and WO 2011/034723 A1 in which the stability of one or both of the volatile delivery enhancing compound and the nicotine or another medicament during storage is maintained. It would also be desirable to provide an aerosol-generating system for delivering nicotine or another medicament to a user of the type disclosed in WO 2008/121610 A1, WO 2010/107613 A1 and WO 2011/034723 A1 in which one or both of the volatile delivery enhancing compound and the nicotine or another medicament is released only upon use of the aerosol-generating system.

US 2012/298123 A1 discloses heat not burn products comprising a heat source and an aerosol generating portion comprising tobacco and an encapsulated aerosol generating agent. US 2012/298123 A1 discloses that the aerosol generating agent may be, for instance, a polyol aerosol generator or a non-polyol aerosol generator and that the aerosol generating agent may be a solid or liquid at room temperature, but preferably is a liquid at room temperature. US 2012/298123 A1 discloses that suitable non-polyols include monohydric alcohols, high boiling point hydrocarbons, acids such as lactic acid, and esters such as diacetin, triacetin, triethyl citrate or isopropyl myristate. US 2012/298123 A1 discloses that the encapsulated aerosol generating agent is encapsulated in a barrier material, which not only provides hindrance to migration during storage of the heat not burn product but allows controlled release of the agent during use. US 2012/298123 A1 discloses that the barrier material may be one that melts, decomposes, reacts, degrades, swells or deforms to release the aerosol generating agent at a temperature above room temperature but at or below the temperature reached inside the heat not burn product during use and that, in embodiments of the invention, the barrier material releases substantial amounts of the aerosol generating agent above 50° C., preferably above 60° C., 70° C., 80° C. or 90° C. US 2012/298123 A1 discloses that, in some embodiments of the invention, the barrier material is applied to particulate carrier material (such as a particulate sorbent material) carrying the aerosol generating agent.

According to the present invention there is provided an aerosol-generating system comprising: an acid source; and a nicotine source, wherein the nicotine source is an encapsulated nicotine source, and wherein the acid source is an encapsulated volatile liquid source comprising: a sorption element; a volatile liquid having a vapour pressure of at least about 20 Pa at 25°C sorbed on the sorption element, wherein the volatile liquid comprises a 2-oxo acid; and a sealant having a melting point of between about 40°C and about 120°C encapsulating the sorption element.

As used herein, by "sorbed" it is meant that the volatile liquid is adsorbed on the surface of the sorption element, or absorbed in the sorption element, or both adsorbed on and absorbed in the sorption element. Preferably, the volatile liquid is adsorbed on the sorption element.

As used herein, by "encapsulating" it is meant the sealant forms a barrier or shell around the sorption element.

The sealant retains the volatile liquid sorbed on the sorption element and so substantially reduces or prevents evaporation and loss of the volatile liquid. This advantageously improves retention of the volatile liquid during storage of encapsulated volatile liquid sources of aerosol-generating systems according to the invention.

The sealant also isolates the sorption element with the volatile liquid sorbed thereon from exposure to external atmospheric effects and so substantially reduces or prevents reaction of the volatile liquid with atmospheric oxygen and water. This advantageously improves the stability of the volatile liquid during storage of encapsulated volatile liquid sources of aerosol-generating systems according to the invention.

Preferably, the sealant forms a barrier or shell around the sorption element that prevents contact of the volatile liquid with the atmosphere. More preferably, the sealant forms a barrier or shell around the sorption element that prevents contact of the volatile liquid with the atmosphere and reduces or prevents exposure of the volatile liquid to light.

Preferably, the sealant forms a barrier or shell around the sorption element that provides an environment for the volatile liquid such that the volatile liquid remains stable upon storage at ambient temperature for a period of at least two months, more preferably for a period of at least four months.

The volatile liquid can be released from the encapsulated volatile liquid source when desired by heating the encapsulated volatile liquid source to a temperature above the melting point of the sealant. Heating the encapsulated volatile liquid source above the melting point of the sealant causes the sealant to melt, thus releasing the volatile liquid sorbed on the sorption element. Encapsulated volatile liquid sources of aerosol-generating systems according to the present invention thus provide a means for temperature controlled release of volatile liquids comprising compounds such as, for example, pyruvic acid and nicotine.

Preferably, the volatile liquid has a vapour pressure of at least about 50 Pa, more preferably at least about 75 Pa, most preferably at least 100 Pa at 25°C. Unless otherwise stated, all vapour pressures referred to herein are vapour pressures at 25°C measured in accordance with ASTM E1194 - 07.

Preferably, the volatile liquid has a vapour pressure of less than or equal to about 400 Pa, more preferably less than or equal to about 300 Pa, even more preferably less than or equal to about 275 Pa, most preferably less than or equal to about 250 Pa at 25°C.

In certain embodiments, the volatile liquid may have a vapour pressure of between about 20 Pa and about 400 Pa, more preferably between about 20 Pa and about 300 Pa, even more preferably between about 20 Pa and about 275 Pa, most preferably between about 20 Pa and about 250 Pa at 25°C.

In other embodiments, the volatile liquid may have a vapour pressure of between about 50 Pa and about 400 Pa, more preferably between about 50 Pa and about 300 Pa, even more preferably between about 50 Pa and about 275 Pa, most preferably between about 50 Pa and about 250 Pa at 25°C.

In further embodiments, the volatile liquid may have a vapour pressure of between about 75 Pa and about 400 Pa, more preferably between about 75 Pa and about 300 Pa, even more preferably between about 75 Pa and about 275 Pa, most preferably between about 75 Pa and about 250 Pa at 25°C.

In yet further embodiments, the volatile liquid may have a vapour pressure of between about 100 Pa and about 400 Pa, more preferably between about 100 Pa and about 300 Pa, even more preferably between about 100 Pa and about 275 Pa, most preferably between about 100 Pa and about 250 Pa at 25°C.

The volatile liquid may comprise a single compound. Alternatively, the volatile compound may comprise two or more different compounds.

Where the volatile liquid comprises two or more different compounds, the two or more different compounds in combination have a vapour pressure of at least about 20 Pa at 25°C.

The volatile liquid may comprise a mixture of two or more different liquid compounds.

The volatile liquid may comprise an aqueous solution of one or more compounds. Alternatively the volatile liquid may comprise a non-aqueous solution of one or more compounds.

The volatile liquid may comprise two or more different volatile compounds. For example, the volatile liquid may comprise a mixture of two or more different volatile liquid compounds.

Alternatively, the volatile liquid may one or more non-volatile compounds and one or more volatile compounds. For example, the volatile liquid may comprise a solution of one or more non-volatile compounds in a volatile solvent or a mixture of one or more non-volatile liquid compounds and one or more volatile liquid compounds.

The volatile liquid comprises an alpha-keto or 2-oxo acid.

In a preferred embodiment, the volatile liquid comprises an acid selected from the group consisting of 3-methyl-2-oxopentanoic acid, pyruvic acid, 2-oxopentanoic acid, 4-methyl-2-oxopentanoic acid, 3-methyl-2-oxobutanoic acid, 2-oxooctanoic acid and combinations thereof. In a particularly preferred embodiment, the volatile liquid comprises pyruvic acid.

The volatile liquid may further comprise other components including, but not limited to, natural flavours, artificial flavours and antioxidants.

Preferably, the sealant has a melting point of between about 40°C and about 100°C, more preferably between about 40°C and about 70°C, most preferably between about 40°C and about 60°C.

Preferably, the sealant has a narrow melting point range. As used herein, the term "melting point range" is used to describe the range between the temperature at which the sealant begins to melt and the temperature at which the sealant has completely melted.

Preferably, the melting point range of the sealant is less than about 10°C, more preferably less than about 5°C. The use of a sealant having a narrow melting point range advantageously enables the volatile liquid sorbed on the sorption element to be released 'on demand' upon heating of the encapsulated volatile liquid source.

The volatile liquid may be released from the encapsulated volatile liquid source at a temperature above the melting temperature of the sealant. For example, the sealant may melt but still maintain a barrier or shell around the volatile liquid until a temperature above the melting temperature of the sealant is reached.

The sealant may be capable of solidifying and re-encapsulating the volatile liquid sorbed on the sorption element on cooling. This is advantageous where it is desired to release only a portion of the volatile liquid sorbed on the sorption element and to retain the remainder of the volatile liquid sorbed on the sorption element for later use.

Preferably, the sealant comprises a wax.

Waxes are typically liphophilic, non-porous and substantially opaque. A sealant comprising a wax may therefore advantageously form a barrier or shell around the sorption element that prevents contact of the volatile liquid with the atmosphere and reduces or prevents exposure of the volatile liquid to light.

The sealant may comprise one or more natural waxes, or one or more synthetic waxes, or a combination of one or more natural waxes and one or more synthetic waxes.

The sealant may comprise one or more animal waxes, one or more mineral waxes, one or more petroleum waxes, one or more polyolefin waxes, one or more vegetable waxes, or any combination thereof.

Suitable animal waxes include, but are not limited to, beeswax.

Suitable petroleum waxes include, but are not limited to, paraffin waxes.

Suitable polyolefin waxes include, but are not limited to, polyethylene waxes and polypropylene waxes.

Suitable mineral waxes include, but are not limited to, montan wax.

Suitable vegetable waxes include but are not limited to, candelilla wax, carnauba wax, castor wax, and soy wax.

Preferably, the sealant comprises one or more waxes selected from the group consisting of beeswax, carnauba wax, candelilla wax, petroleum waxes, polyolefin waxes, and derivatives thereof.

In a particularly preferred embodiment, the sealant comprises a paraffin wax.

The sorption element may be formed from any suitable material or combination of materials. For example, the sorption element may comprise one or more of glass, stainless steel, aluminium, polyethylene (PE), polypropylene, polyethylene terephthalate (PET), polybutylene terephthalate (PBT), polytetrafluoroethylene (PTFE), expanded polytetrafluoroethylene (ePTFE), and BAREX®.

In a preferred embodiment, the sorption element is a porous sorption element.

For example, the sorption element may be a porous sorption element comprising one or more materials selected from the group consisting of porous plastic materials, porous polymer fibres and porous glass fibres.

The sorption element is preferably chemically inert with respect to the volatile liquid.

The sorption element is preferably physically stable at the melting temperature of the sealant.

The sorption element is preferably physically stable at the temperature at which the volatile liquid is released from the encapsulated volatile liquid source.

The sorption element may have any suitable size and shape. In a preferred embodiment, the sorption element is a substantially cylindrical plug. In a particularly preferred embodiment, the sorption element is a porous substantially cylindrical plug.

The size, shape and composition of the sorption element may be chosen to allow a desired amount of volatile liquid to be sorbed on the sorption element.

In a preferred embodiment, between about 20 µl and about 200 µl, more preferably between about 40 µl and about 150 µl, most preferably between about 50 µl and about 100 µl of the volatile liquid is sorbed on the sorption element.

The sorption element advantageously acts as a reservoir for the volatile liquid.

The sorption element may be encapsulated with the sealant by any suitable method.

For example, the sealant may be melted, the sorption element coated with the molten sealant and the molten sealant then solidified in order to encapsulate the sorption element with the sealant. The sorption element may be coated with the molten sealant by any suitable method, such as spraying, painting or fluidized bed coating.

Alternatively, the sealant may be melted, the sorption element dipped in the molten sealant and the molten sealant then solidified in order to encapsulate the sorption element with the sealant.

Depending on the properties of the sealant, an active cooling step may be implemented to solidify the sealant at a faster rate. The inclusion of an active cooling step may advantageously result in a more even and uniform coating by reducing creep of the semi-solid sealant under gravity and by reducing penetration of the sealant into the sorption element. The active cooling step may comprise contacting the sealant with a gaseous cooling agent or a liquid cooling agent. For example, the molten sealant may be dipped in a bath of liquid cooling agent, such as liquid nitrogen or chilled isopropyl alcohol, or the molten sealant may be cooled in a stream of gaseous cooling agent, such as cold air.

The volatile liquid may be sorbed on the sorption element before the sorption element is encapsulated by the sealant.

Alternatively, the sorption element may be encapsulated by the sealant, the volatile liquid injected through the sealant onto or into the sorption element and the injection site then sealed.

In a particularly preferred embodiment, there is provided an aerosol-generating system comprising a housing, the housing comprising: a) an inlet and an outlet in communication with each other and adapted so that a gaseous carrier may pass into the housing through the inlet, through the housing and out of the housing through the outlet, the aerosol-generating system comprising in series from inlet to outlet: b) a first internal area in communication with the inlet, the first internal area comprising a first one of an acid source and a nicotine source, and c) a second internal area in communication with the first internal area, the second internal area comprising a second one of the acid source and the nicotine source, wherein the acid source is an encapsulated volatile liquid source comprising: a sorption element; a volatile liquid having a vapour pressure of at least about 20 Pa at 25°C sorbed on the sorption element, wherein the volatile liquid comprises a 2-oxo acid; and a sealant having a melting point of between about 40°C and about 120°C encapsulating the sorption element.

The aerosol-generating system may further comprise a third internal area in communication with: the second internal area; and the outlet.

The aerosol-generating system may further comprise a mouthpiece in communication with: the second internal area, or the third internal area, where present; and the outlet.

In another preferred embodiment, there is provided an aerosol-generating system comprising a housing, the housing comprising: a) an inlet and an outlet in communication with each other and adapted so that a gaseous carrier may pass into the housing through the inlet, through the housing and out of the housing through the outlet, the aerosol-generating system comprising in parallel: b) a first internal area in communication with the inlet, the first internal area comprising an acid source, and c) a second internal area in communication with the inlet, the second internal area comprising a nicotine source, wherein the acid source is an encapsulated volatile liquid source comprising: a sorption element; a volatile liquid having a vapour pressure of at least about 20 Pa at 25°C sorbed on the sorption element, wherein the volatile liquid comprises a 2-oxo acid; and a sealant having a melting point of between about 40°C and about 120°C encapsulating the sorption element. In use, a first stream of the gaseous carrier passes through the first internal area and a second stream of the gaseous carrier passes through the second internal area.

The aerosol-generating system may further comprise a third internal area in communication with: one or both of the first internal area and the second internal area; and the outlet.

The aerosol-generating system may further comprise a mouthpiece in communication with: the first internal area and the second internal area, or the third internal area, where present; and the outlet.

In a further preferred embodiment, there is provided an aerosol-generating system comprising a housing, the housing comprising: a) a first air inlet, a second air inlet and an outlet, the first air inlet and the second air inlet in communication with the outlet and adapted so that a gaseous carrier may pass into the housing through the first air inlet, through the housing and out of the housing through the outlet and a gaseous carrier may pass into the housing through the second air inlet, through the housing and out of the housing through the outlet, the aerosol-generating system comprising in parallel: b) a first internal area in communication with the first inlet, the first internal area comprising an acid source, and c) a second internal area in communication with the second air inlet, the second internal area comprising a nicotine source, wherein the acid source is an encapsulated volatile liquid source comprising: a sorption element; a volatile liquid having a vapour pressure of at least about 20 Pa at 25°C sorbed on the sorption element, wherein the volatile liquid comprises a 2-oxo acid; and a sealant having a melting point of between about 40°C and about 120°C encapsulating the sorption element.

The aerosol-generating system may further comprise a third internal area in communication with: one or both of the first internal area and the second internal area; and the outlet.

The aerosol-generating system may further comprise a mouthpiece in communication with: the first internal area and the second internal area, or the third internal area, where present; and the outlet.

The nicotine source of aerosol-generating systems according to the invention is an encapsulated nicotine source. The encapsulated nicotine source preferably comprises: a sorption element; a volatile liquid having a vapour pressure of at least about 20 Pa at 25°C sorbed on the sorption element and a sealant having a melting point of between about 40°C and about 120°C encapsulating the sorption element. Suitable sorption elements and sealants for use in such encapsulated nicotine sources are described above.

The nicotine source may comprise one or more of nicotine, nicotine base, a nicotine salt, such as nicotine-HCI, nicotine-bitartrate, or nicotine-ditartrate, or a nicotine derivative.

The nicotine source may comprise natural nicotine or synthetic nicotine.

The nicotine source may comprise pure nicotine, a solution of nicotine in an aqueous or non-aqueous solvent or a liquid tobacco extract.

The nicotine source may further comprise an electrolyte forming compound. The electrolyte forming compound may be selected from the group consisting of alkali metal hydroxides, alkali metal oxides, alkali metal salts, alkaline earth metal oxides, alkaline earth metal hydroxides and combinations thereof.

For example, the nicotine source may comprise an electrolyte forming compound selected from the group consisting of potassium hydroxide, sodium hydroxide, lithium oxide, barium oxide, potassium chloride, sodium chloride, sodium carbonate, sodium citrate, ammonium sulfate and combinations thereof.

In certain embodiments, the nicotine source may comprise an aqueous solution of nicotine, nicotine base, a nicotine salt or a nicotine derivative and an electrolyte forming compound.

Where aerosol-generating systems according to the invention comprise a third internal area, the third internal area may comprise one or more aerosol-modifying agents. For example, the third internal area may comprise a sorbent, such as activated carbon, a flavourant, such as menthol, or a combination thereof.

Where aerosol-generating systems according to the invention comprise a mouthpiece, the mouthpiece may comprise a filter. The filter may have a low particulate filtration efficiency or very low particulate filtration efficiency.

In a preferred embodiment, there is provided an aerosol-generating system comprising: an acid source; and a nicotine source, wherein the nicotine source is an encapsulated nicotine source, and wherein the acid source is an encapsulated volatile liquid source comprising a sorption element, a volatile liquid having a vapour pressure of at least about 20 Pa at 25°C sorbed on the sorption element, wherein the volatile liquid comprises a 2-oxo acid, and a sealant having a melting point of between about 40°C and about 120°C encapsulating the sorption element; and heating means for heating the encapsulated volatile liquid source to a temperature above the melting point of the sealant.

In certain preferred embodiments, the aerosol-generating system may comprise: an aerosol-generating article comprising an acid source; and a nicotine source, wherein the nicotine source is an encapsulated nicotine source, and wherein the acid source is an encapsulated volatile liquid source comprising a sorption element, a volatile liquid having a vapour pressure of at least about 20 Pa at 25°C sorbed on the sorption element, wherein the volatile liquid comprises a 2-oxo acid, and a sealant having a melting point of between about 40°C and about 120°C encapsulating the sorption element; and an aerosol-generating device in cooperation with the aerosol-generating article, the aerosol generating device comprising heating means for heating the encapsulated volatile liquid source to a temperature above the melting point of the sealant.

The heating means may be an electric heating means comprising an electric heater powered by an electric power supply. Where the heating means is an electric heating means, the aerosol-generating system may further comprise an electric power supply, such as a battery, and electronic circuitry configured to control the supply of electric power from the electric power supply to the electric heating means.

Alternatively, the heating means may be a non-electric heating means, such as a chemical heating means.

Aerosol-generating systems according to the invention may simulate a smoking article, such as a cigarette, a cigar, a cigarillo or a pipe, or a cigarette pack. In preferred embodiments, aerosol-generating systems according to the invention simulate a cigarette.

In certain embodiments, the housing of aerosol-generating systems according to the invention may simulate a tobacco smoking article, such as a cigarette, a cigar, a cigarillo or a pipe, or a cigarette pack. In certain preferred embodiments, the housing simulates a cigarette.

For the avoidance of doubt, features described above in relation to one aspect of the invention may also be applicable to other aspects of the invention.

Use of an encapsulated volatile liquid source in an aerosol-generating system according to the invention comprising an acid source and a nicotine source provides a means to control delivery of aerosolised nicotine salt particles, such as nicotine pyruvate particles, to a user.

The invention will now be further described with reference to the accompanying drawings in which:
Figures 1 shows a Fourier transform infrared spectrum at a wavelength of 1810cm⁻¹ for a pyruvic acid source according to the invention upon heating as a function of temperature;
Figure 2 shows the percentage of pyruvic acid remaining in a pyruvic acid source according to the invention versus time over a period of 30 days; and
Figure 3 shows the pyruvic acid yield and average puff temperature for a pyruvic acid source according to the invention upon heating measured under a Health Canada smoking regime.

### Example 1

50 µl of pyruvic acid is adsorbed on a sintered porous plastic plug with a density of 0.3g/cc having a polyethylene terephthalate (PET) core, a polyethylene (PE) sheath and a viscose B fibre filling. A suitable porous plastic plug is Porex® XMF-0130+B (available from Porex GmbH, Germany). The porous plastic plug with the 50 µl of pyruvic acid adsorbed thereon is encapsulated in a paraffin wax with a melting point of 54-56°C by melting the paraffin wax, dipping the porous plastic plug into the molten paraffin wax and then solidifying the molten paraffin wax. A heating ramp of 3°C/minute is applied to the resulting porous plastic plug with the 50 µl of pyruvic acid adsorbed thereon encapsulated in the paraffin wax and the vapours released are analysed by Fourier transform infrared (FTIR) spectroscopy.

For comparison, a heating ramp of 3°C/minute is also applied to an identical porous plastic plug with 50 µl of pyruvic acid adsorbed thereon that is not encapsulated in a paraffin wax and the vapours released are analysed by FTIR spectroscopy.

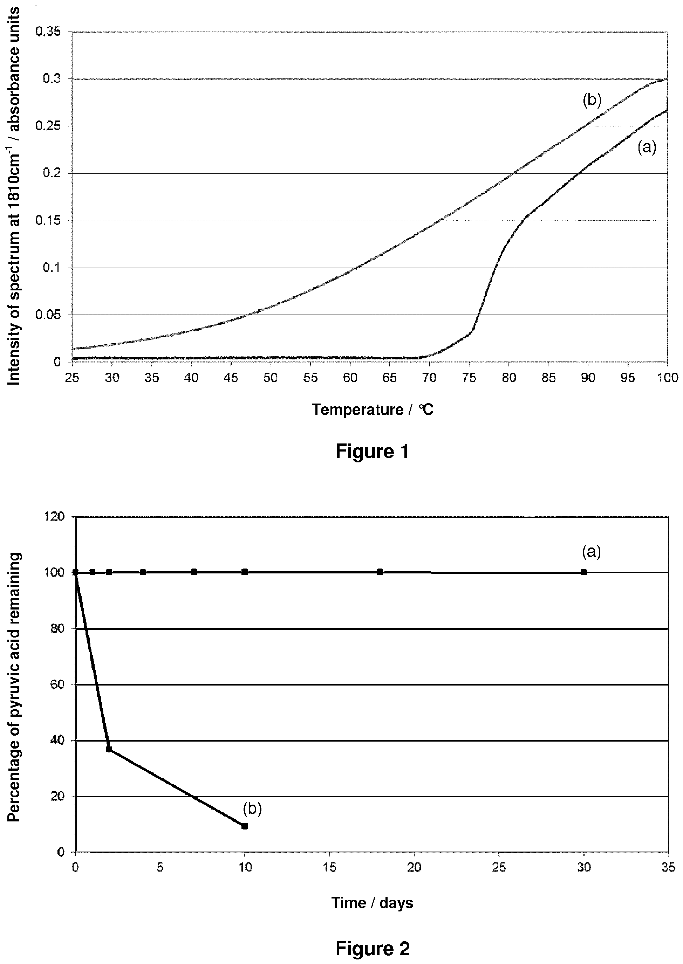

Figure 1 shows the FTIR spectra at a wavelength of 1810cm1, corresponding to the characteristic spectra associated with pyruvic acid, for (a) the porous plastic plug with the 50 µl of pyruvic acid adsorbed thereon encapsulated in the paraffin wax, and (b) the porous plastic plug with 50 µl of pyruvic acid adsorbed thereon that is not encapsulated in a paraffin wax.

As shown in Figure 1, the porous plastic plug with the 50 µl of pyruvic acid adsorbed thereon encapsulated in the paraffin wax shows no release of pyruvic acid until approximately 70°C. In contrast, the porous plastic plug with 50 µl of pyruvic acid adsorbed thereon that is not encapsulated in a paraffin wax releases pyruvic acid immediately on heating.

### Example 2

Example 1 is repeated using beeswax rather than paraffin wax.

A heating ramp of 3°C/minute is applied to the resulting porous plastic plug with the 50 µl of pyruvic acid adsorbed thereon encapsulated in the beeswax and the vapours released are analysed by Fourier transform infrared (FTIR) spectroscopy.

For comparison, a heating ramp of 3°C/minute is also applied to an identical porous plastic plug with 50 µl of pyruvic acid adsorbed thereon that is not encapsulated in beeswax and the vapours released are analysed by FTIR spectroscopy.

The porous plastic plug with the 50 µl of pyruvic acid adsorbed thereon encapsulated in the beeswax shows no release of pyruvic acid until approximately 80°C. In contrast, the porous plastic plug with 50 µl of pyruvic acid adsorbed thereon that is not encapsulated in beeswax releases pyruvic acid immediately on heating.

### Example 3

Example 1 is repeated using carnauba wax rather than paraffin wax.

A heating ramp of 3°C/minute is applied to the resulting porous plastic plug with the 50 µl of pyruvic acid adsorbed thereon encapsulated in the carnauba wax and the vapours released are analysed by Fourier transform infrared (FTIR) spectroscopy.

For comparison, a heating ramp of 3°C/minute is also applied to an identical porous plastic plug with 50 µl of pyruvic acid adsorbed thereon that is not encapsulated in carnauba wax and the vapours released are analysed by FTIR spectroscopy.

The porous plastic plug with the 50 µl of pyruvic acid adsorbed thereon encapsulated in the carnauba wax shows no release of pyruvic acid until approximately 100°C. In contrast, the porous plastic plug with 50 µl of pyruvic acid adsorbed thereon that is not encapsulated in carnauba wax releases pyruvic acid immediately on heating.

### Example 4

50 µl of pyruvic acid is adsorbed on a sintered porous plastic plug with a density of 0.17g/cc having a polyethylene terephthalate (PET) core and a polyethylene (PE) sheath. A suitable porous plastic plug is Porex® XMF-0507 (available from Porex GmbH, Germany). The porous plastic plug with the 50 µl of pyruvic acid adsorbed thereon is encapsulated in a paraffin wax with a melting point of 54-56°C by melting the paraffin wax, dipping the porous plastic plug into the molten paraffin wax and then solidifying the molten paraffin wax. The resulting porous plastic plug with the 50 µl of pyruvic acid adsorbed thereon encapsulated in the paraffin wax is stored at 22°C and 50% relative humidity and its mass is measured over a period of 30 days.

For comparison, an identical porous plastic plug with 50 µl of pyruvic acid adsorbed thereon that is not encapsulated in a paraffin wax is also stored at 22°C and 50% relative humidity and its mass is measured over a period of 30 days.

Figure 2 shows the percentages of pyruvic acid remaining in (a) the porous plastic plug with the 50 µl of pyruvic acid adsorbed thereon encapsulated in the paraffin wax, and (b) the porous plastic plug with 50 µl of pyruvic acid adsorbed thereon that is not encapsulated in a paraffin wax, estimated by the total mass loss.

As shown in Figure 2, the porous plastic plug with the 50 µl of pyruvic acid adsorbed thereon encapsulated in the paraffin wax does not lose any of the pyruvic acid adsorbed thereon over the 30 day period. In contrast, the porous plastic plug with 50 µl of pyruvic acid adsorbed thereon that is not encapsulated in a paraffin wax loses over 80% of the pyruvic acid adsorbed thereon in 10 days.

### Example 5

50 µl of pyruvic acid is adsorbed on a on a sintered porous plastic plug with a density of 0.17g/cc having a polyethylene terephthalate (PET) core and a polyethylene (PE) sheath. A suitable porous plastic plug is Porex® XMF-0507 (available from Porex GmbH, Germany). The porous plastic plug with the 50 µl of pyruvic acid adsorbed thereon is encapsulated in a paraffin wax with a melting point of 54-56°C by melting the paraffin wax, dipping the porous plastic plug into the molten paraffin wax and then solidifying the molten paraffin wax.

The resulting porous plastic plug with the 50 µl of pyruvic acid adsorbed thereon encapsulated in the paraffin wax is heated to 75°C and the pyruvic acid yield per group of five puffs is measured under a Health Canada smoking regime over 20 puffs with a puff volume of 55 ml, puff duration of 2 seconds and a puff interval of 30 seconds.

An identical porous plastic plug with 50 µl of pyruvic acid adsorbed thereon encapsulated in the paraffin wax is stored at 22°C and 50% relative humidity for 30 days and is then heated to 75°C and the pyruvic acid yield per group of five puffs is measured under a Health Canada smoking regime over 20 puffs with a puff volume of 55 ml, puff duration of 2 seconds and a puff interval of 30 seconds.

For comparison, an identical porous plastic plug with the 50 µl of pyruvic acid adsorbed thereon that is not encapsulated in a paraffin wax is heated to 75°C and the pyruvic acid yield per group of five puffs is measured under a Health Canada smoking regime over 20 puffs with a puff volume of 55 ml, puff duration of 2 seconds and a puff interval of 30 seconds.

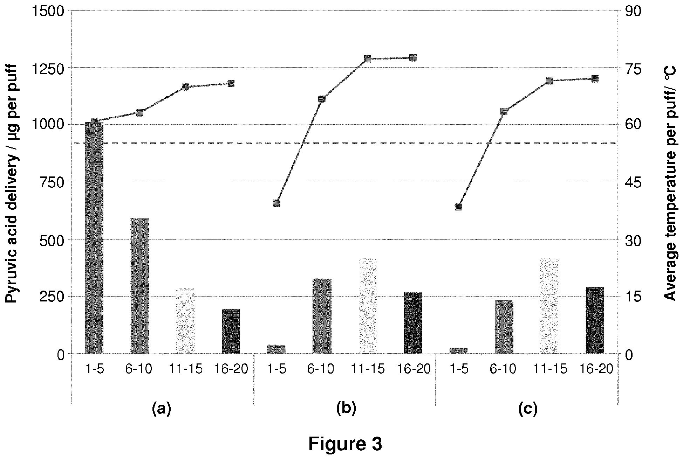

Figure 3 shows the pyruvic acid yield and average puff temperature per group of five puffs for (a) the porous plastic plug with 50 µl of pyruvic acid adsorbed thereon that is not encapsulated in a paraffin wax, (b) the porous plastic plug with the 50 µl of pyruvic acid adsorbed thereon encapsulated in the paraffin wax, and (c) the porous plastic plug with the 50 µl of pyruvic acid adsorbed thereon encapsulated in the paraffin wax and stored for 30 days.

As shown in Figure 3, the yield of pyruvic acid in the first group of five puffs for (b) the porous plastic plug with the 50 µl of pyruvic acid adsorbed thereon encapsulated in the paraffin wax, and (c) the porous plastic plug with the 50 µl of pyruvic acid adsorbed thereon encapsulated in the paraffin wax and stored for 30 days is less than that for (a) the porous plastic plug with 50 µl of pyruvic acid adsorbed thereon that is not encapsulated in a paraffin wax. This is because pyruvic acid is only released from (b) the porous plastic plug with the 50 µl of pyruvic acid adsorbed thereon encapsulated in the paraffin wax, and (c) the porous plastic plug with the 50 µl of pyruvic acid adsorbed thereon encapsulated in the paraffin wax and stored for 30 days once the melting point of the paraffin wax (shown by the dashed line in Figure 3) is reached, which does not occur until about fourth puff. As also shown in Figure 3, the yield of pyruvic acid for (c) the porous plastic plug with the 50 µl of pyruvic acid adsorbed thereon encapsulated in the paraffin wax and stored for 30 days is similar to that of (b) the porous plastic plug with the 50 µl of pyruvic acid adsorbed thereon encapsulated in the paraffin wax. This indicates that the pyruvic acid is stored durably on the porous plastic plug encapsulated in the paraffin wax over the 30 day period.

As illustrated by the above examples, in accordance with the present invention it is possible to control the delivery of a volatile liquid comprising a 2-oxo acid, such as pyruvic acid, by controlling the encapsulation of a sorption element on which the volatile liquid is sorbed. Using a sealant, such as but not limited to a wax such as for example a paraffin wax, of certain melting point for encapsulating a sorption element, such as but not limited to a porous plastic plug, having the volatile liquid sorbed thereon provides a means for temperature controlled delivery of the volatile liquid.

The invention has been exemplified above by reference to porous plastic plugs, such as Porex® plugs having pyruvic acid adsorbed thereon that are encapsulated in a paraffin wax, beeswax or carnauba wax. However, it will be appreciated that encapsulated volatile liquid sources according to the invention may comprise other adsorption elements, other volatile liquids comprising a 2-oxo acid and other sealants.

## Claims

1. An aerosol-generating system comprising:
an acid source; and
a nicotine source,
wherein the nicotine source is an encapsulated nicotine source, and
wherein the acid source is an encapsulated volatile liquid source comprising:
a sorption element;
a volatile liquid having a vapour pressure of at least about 20 Pa at 25°C sorbed on the sorption element, wherein the volatile liquid comprises a 2-oxo acid; and
a sealant having a melting point of between about 40°C and about 120°C encapsulating the sorption element.

2. An aerosol-generating system according to claim 1 wherein the volatile liquid has a vapour pressure of at least about 50 Pa at 25°C.

3. An aerosol-generating system according to claim 1 or 2 wherein the acid is selected from the group consisting of 3-methyl-2-oxovaleric acid, pyruvic acid, 2-oxovaleric acid, 4-methyl-2-oxovaleric acid, 3-methyl-2-oxobutanoic acid, 2-oxooctanoic acid and combinations thereof.

4. An aerosol-generating system according to claim 3 wherein the acid is pyruvic acid.

5. An aerosol-generating system according to any preceding claim wherein the sealant has a melting point of between about 40°C and about 70°C.

6. An aerosol-generating system according to any preceding claim wherein the sealant comprises a wax.

7. An aerosol-generating system according to claim 6 wherein the sealant comprises one or more waxes selected from the group consisting of beeswax, carnauba wax, candelilla wax, petroleum waxes, polyolefin waxes, and derivatives thereof.

8. An aerosol-generating system according to claim 7 wherein the sealant comprises a paraffin wax.

9. An aerosol-generating system according to any preceding claim wherein the sorption element is a porous adsorption element

10. An aerosol-generating system according to any preceding claim further comprising:
heating means for heating the encapsulated volatile liquid source to a temperature above the melting point of the sealant

11. An aerosol-generating system according to any preceding claim wherein the aerosol-generating system simulates a cigarette.

12. An aerosol-generating system according to any preceding claim wherein the encapsulated nicotine source comprises:
a sorption element;
a volatile liquid having a vapour pressure of at least about 20 Pa at 25°C sorbed on the sorption element; and
a sealant having a melting point of between about 40°C and about 120°C encapsulating the sorption element.

## Patentansprüche

1. Aerosolerzeugungssystem, aufweisend:
eine Säurequelle; und
eine Nikotinquelle,
wobei die Nikotinquelle eine eingekapselte Nikotinquelle ist, und
wobei die Säurequelle eine eingekapselte flüchtige Flüssigkeitsquelle ist, aufweisend:
ein Sorptionselement;
eine flüchtige Flüssigkeit mit einem Dampfdruck von mindestens ungefähr 20 Pa bei 25 °C, die an das Sorptionselement sorbiert ist, wobei die flüchtige Flüssigkeit eine 2-Oxosäure aufweist; und
ein Dichtungsmittel mit einem Schmelzpunkt zwischen ungefähr 40 °C und ungefähr 120 °C, welches das Sorptionselement einkapselt.

2. Aerosolerzeugungssystem nach Anspruch 1, wobei die flüchtige Flüssigkeit einen Dampfdruck von mindestens ungefähr 50 Pa bei 25 °C aufweist.

3. Aerosolerzeugungssystem nach Anspruch 1 oder 2, wobei die Säure ausgewählt ist aus der Gruppe bestehend aus 3-Methyl-2-oxovaleriansäure, Pyruvinsäure, 2-Oxovaleriansäure, 4-Methyl-2-oxovaleriansäure, 3-Methyl-2-oxobutansäure, 2-Oxooctansäure und Kombinationen davon.

4. Aerosolerzeugungssystem nach Anspruch 3, wobei die Säure Pyruvinsäure ist.

5. Aerosolerzeugungssystem nach einem der vorstehenden Ansprüche, wobei das Dichtungsmittel einen Schmelzpunkt zwischen ungefähr 40 °C und ungefähr 70 °C aufweist.

6. Aerosolerzeugungssystem nach einem der vorstehenden Ansprüche, wobei das Dichtungsmittel ein Wachs aufweist.

7. Aerosolerzeugungssystem nach Anspruch 6, wobei das Dichtungsmittel ein oder mehrere Wachse aufweist, die ausgewählt sind aus der Gruppe bestehend aus Bienenwachs, Carnaubawachs, Candelillawachs, Erdölwachsen, Polyolefinwachsen und Derivaten davon.

8. Aerosolerzeugungssystem nach Anspruch 7, wobei das Dichtungsmittel ein Paraffinwachs aufweist.

9. Aerosolerzeugungssystem nach einem der vorstehenden Ansprüche, wobei das Sorptionselement ein poröses Adsorptionselement ist.

10. Aerosolerzeugungssystem nach einem der vorstehenden Ansprüche, weiter aufweisend:
Heizmittel zum Erwärmen der eingekapselten flüchtigen Flüssigkeitsquelle auf eine Temperatur oberhalb des Schmelzpunkts des Dichtungsmittels.

11. Aerosolerzeugungssystem nach einem der vorstehenden Ansprüche, wobei das Aerosolerzeugungssystem eine Zigarette simuliert.

12. Aerosolerzeugungssystem nach einem der vorstehenden Ansprüche, wobei die eingekapselte Nikotinquelle aufweist:
ein Sorptionselement;
eine flüchtige Flüssigkeit mit einem Dampfdruck von mindestens ungefähr 20 Pa bei 25 °C, die an das Sorptionselement sorbiert ist; und
ein Dichtungsmittel mit einem Schmelzpunkt zwischen ungefähr 40 °C und ungefähr 120 °C, welches das Sorptionselement einkapselt.

## Revendications

1. Système de génération d'aérosol, comprenant :
une source d'acide ; et
une source de nicotine,
dans lequel la source de nicotine est une source de nicotine encapsulée, et
dans lequel la source d'acide est une source de liquide volatile encapsulée comprenant :
un élément de sorption ;
un liquide volatil ayant une pression de vapeur d'au moins 20 Pa à 25 °C sorbé sur l'élément de sorption, dans lequel le liquide volatil comprend un acide 2-oxo ; et
un enduit ayant un point de fusion entre environ 40 °C et environ 120 °C encapsulant l'élément de sorption.

2. Système de génération d'aérosol selon la revendication 1, dans lequel le liquide volatile a une pression de vapeur d'au moins 50 Pa à 25 °C.

3. Système de génération d'aérosol selon la revendication 1 ou 2, dans lequel l'acide est choisi dans le groupe constitué de l'acide 3-méthyl-2-oxovalérique, de l'acide pyruvique, de l'acide 2-oxovalérique, de l'acide 4-méthyl-2-oxovalérique, de l'acide 3-méthyle-2-oxobutanoïque, de l'acide 2-oxooctanoïque et leurs combinaisons.

4. Système de génération d'aérosol selon la revendication 3, dans lequel l'acide est l'acide pyruvique.

5. Système de génération d'aérosol selon l'une quelconque des revendications précédentes, dans lequel l'enduit a un point de fusion entre environ 40 °C et environ 70 °C.

6. Système de génération d'aérosol selon l'une quelconque des revendications précédentes, dans lequel l'enduit comprend une cire.

7. Système de génération d'aérosol selon la revendication 6, dans lequel l'enduit comprend un ou plusieurs cires sélectionnés parmi le groupe constitué de cire d'abeille, de cire de carnauba, de cire de candelilla, de cires de pétrole, de cires de polyoléfine et de dérivés de ceux-ci.

8. Système de génération d'aérosol selon la revendication 7, dans lequel l'enduit comprend une cire de paraffine.

9. Système de génération d'aérosol selon l'une quelconque des revendications précédentes, dans lequel l'élément de sorption est un élément d'adsorption poreux.

10. Système de génération d'aérosol selon l'une quelconque des revendications précédentes, comprenant en outre :
des moyens de chauffage pour chauffer la source de liquide volatil encapsulée à une température au-dessus du point de fusion de l'enduit.

11. Système de génération d'aérosol selon l'une quelconque des revendications précédentes, dans lequel le système de génération d'aérosol simule une cigarette.

12. Système de génération d'aérosol selon l'une quelconque des revendications précédentes, dans lequel la source de nicotine encapsulée comprend :
un élément de sorption ;
un liquide volatil ayant une pression de vapeur d'au moins 20 Pa à 25 °C sorbé sur l'élément de sorption ; et
un enduit ayant un point de fusion entre environ 40 °C et environ 120 °C encapsulant l'élément de sorption.
